(19) **Europäisches Patentamt**
**European Patent Office**
**Office européen des brevets**

(11) **EP 4 691 359 A1**

(12) **EUROPEAN PATENT APPLICATION**
published in accordance with Art. 153(4) EPC

(43) Date of publication:
**11.02.2026 Bulletin 2026/07**

(21) Application number: **23932225.8**

(22) Date of filing: **30.10.2023**

(51) International Patent Classification (IPC):
**A61B 5/055** (2006.01)   **A61B 5/00** (2006.01)
**G16H 30/20** (2018.01)   **G16H 30/40** (2018.01)
**G16H 50/20** (2018.01)

(52) Cooperative Patent Classification (CPC):
**A61B 5/00; A61B 5/055; G16H 30/20; G16H 30/40; G16H 50/20**

(86) International application number:
**PCT/KR2023/017006**

(87) International publication number:
**WO 2024/210291 (10.10.2024 Gazette 2024/41)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **04.04.2023 KR 20230044318**

(71) Applicant: **Phantomics Inc.**
**Seoul 07788 (KR)**

(72) Inventor: **KIM, Pan Ki**
**Incheon 22874 (KR)**

(74) Representative: **Laine IP Oy**
**Porkkalankatu 24**
**00180 Helsinki (FI)**

(54) **MRI PROCESSING DEVICE AND METHOD, AND COMPUTER-READABLE RECORDING MEDIUM STORING PROGRAM FOR PERFORMING METHOD**

(57) Disclosed are a magnetic resonance imaging (MRI) processing device and method, and a computer-readable recording medium storing a program for performing the method. An MRI processing device according to an exemplary embodiment of the present invention is an MRI processing device for generating late gadolinium enhancement (LGE) magnetic resonance imaging (MRI), and may comprise: a storage medium for storing extracellular volume (ECV), a hematocrit level in blood, a T1 value of a heart muscle before contrast agent injection, a T1 value of blood before contrast agent injection, and a T1 value of blood after contrast agent injection; and a processor that, on the basis of a correlation between the ECV, the hematocrit level in the blood, the T1 value of the heart muscle before contrast agent injection, the T1 value of the blood before contrast agent injection, and the T1 value of the blood after contrast agent injection, calculates a T1 value of the heart muscle after contrast agent injection, and calculates an optimal inversion time for MRI using the T1 value of the heart muscle after contrast agent injection.

[FIG.1]

100

STORAGE MEDIUM —— 110

PROCESSOR —— 120

**Description**

TECHNICAL FIELD

The present invention relates to an MRI processing device and method, and a computer-readable recording medium storing a program for performing the method, and more particularly, to an MRI processing device and method for generating late gadolinium enhancement (LGE) MRI, and a computer-readable recording medium storing a program for performing the method.

BACKGROUND ART

Magnetic resonance imaging (MRI) refers to a diagnostic test that transmits RF (radio frequency) pulses to a subject's human body while the subject is located within a strong magnetic field and measures the reflected magnetic field to obtain an image. The advantage of MRI is that it uses RF pulses and magnetic fields, so it is harmless to the human body compared to X-rays or CT using radiation, and can obtain informat ion on soft tissues in various ways.

Among various MRI techniques, late gadolinium enhancement (LGE) MRI is effective in diagnosing myocardial lesions. Late gadolinium enhancement MRI takes advantage of the tendency of contrast agent to stay longer in intramyocardial lesion than in normal myocardium. In more detail, late gadolinium enhancement MRI allows us to determine the presence or absence of a lesion based on the difference in the intensity of the imaging signal between the normal myocardium and the lesion shown in the MRI data obtained in the time range where a relatively large amount of the contrast agent in the normal myocardium is discharged and a relatively large amount of the contrast agent stays in the lesion after the contrast agent is injected.

After the contrast agent is injected, a relatively large amount of the contrast agent in the normal myocardium is discharged, and the time when the contrast enhancement effects appear due to a relatively large amount of the contrast agent staying in the lesion is as short as a few seconds. In addition, the intensity of the imaging signal of the normal myocardium and lesion that appear on MRI for a short period of time within a few seconds is constantly changing.

For accurate determination of the lesion, MRI images are needed at a time point when the difference in shade between the normal myocardium and the lesion is above a certain level during a short period of time when a relatively large amount of contrast agent is discharged and a large amount of contrast agent remains in the lesion. However, there is still no technology to accurately capture and image a time point when the difference in the shadow between the normal myocardium and the lesion on MRI appears above a certain level during a relatively large amount of contrast agent in the normal myocardium is discharged and a relatively large amount of contrast agent remains in the lesion.

(Patent Document) Korean Patent Laid-Open Publication No. 10-2022-0030896, "Method for correcting magnetic resonance imaging error using heart rate interval", published March 11, 2022.

DISCLOSURE

TECHNICAL PROBLEM

The present invention is directed to providing an MRI processing device and method that allow us to automatically calculate a time point when the difference in shadow between normal tissue and lesion appears above a certain level in late gadolinium enhancement (LGE) MRI, and obtain MRI images at that time point through image post-processing using a post T1 map, and a computer-readable recording medium storing a program for performing the method.

The problems of the present invention are not limited to those mentioned above, and other problems not mentioned will be clearly understood by those of ordinary skill in the art from the following description.

TECHNICAL SOLUTION

According to an aspect of the present invention, provided is an MRI processing device configured to generate late gadolinium enhancement (LGE) MRI (magnetic resonance imaging), the MRI processing device including: a storage medium configured to store an extracellular volume (ECV), a hematocrit level in blood, a T1 value of a heart muscle before contrast agent injection, a T1 value of blood before contrast agent injection, and a T1 value of blood after contrast agent injection; and a processor configured to calculate a T1 value of a heart muscle after contrast agent injection, and calculate an optimal inversion time for MRI using the T1 value of a heart muscle after contrast agent injection, based on a correlation between the ECV, the hematocrit level in blood, the T1 value of a heart muscle before contrast agent injection, the T1 value of blood before contrast agent injection, and the T1 value of blood after contrast agent injection.

In the MRI processing device according to an aspect of the present invention, the processor may calculate the T1 value of a heart muscle after contrast agent injection, based on Mathematical Equation 1 below:

(Mathematical Equation 1)

$$\frac{1}{T1\_Post\_Myo} = ECV \times \frac{\frac{1}{T1\_Post\_Blood} - \frac{1}{T1\_Pre\_Blood}}{1-HCT} - \frac{1}{T1\_Pre\_Myo}$$

(T1_Post_Myo: a T1 value of a heart muscle after contrast agent injection, HCT: a hematocrit level in blood, T1_Pre_Myo: a T1 value of a heart muscle before contrast agent injection, T1_Pre_Blood: a T1 value of blood before contrast agent injection, T1_Post_Blood: a T1 value of blood after contrast agent injection).

In the MRI processing device according to an aspect of the present invention, the ECV may have a value selected in the range of 24 to 28%.

In the MRI processing device according to an aspect of the present invention, the hematocrit level in blood may have a value selected in the range of 38 to 42%.

In the MRI processing device according to an aspect of the present invention, the processor may calculate the optimal inversion time by substituting the T1 value of a heart muscle after contrast agent injection into Mathematical Equation 2 below:

(Mathematical Equation 2)

$$TI\_optimal = 0.69 \times T1\_Post\_Myo$$

(TI_optimal: optimal inversion time, T1_Post_Myo: a T1 value of a heart muscle after contrast agent injection).

In the MRI processing device according to an aspect of the present invention, the processor may generate a normalized imaging signal by normalizing an imaging signal included in the MRI data at the optimal inversion time using Mathematical Equation 3 below:

(Mathematical Equation 3)

$$S\_normal = A \times S\_TI\_optimal$$

(S_normal: normalized imaging signal, S_TI_optimal: imaging signal included in MRI data at optimal inversion time, A is a real number greater than or equal to 1).

In the MRI processing device according to an aspect of the present invention, the processor may determine a portion where the normalized MRI signal is greater than or equal to a reference value on the MRI at the optimal inversion time as a lesion.

According to another aspect of the present invention, provided is an MRI processing method for generating late gadolinium enhancement (LGE) MRI (magnetic resonance imaging), the MRI processing method including: storing, by a storage medium, an extracellular volume (ECV), a hematocrit level in blood, a T1 value of a heart muscle before contrast agent injection, a T1 value of blood before contrast agent injection, and a T1 value of blood after contrast agent injection; and calculating, by a processor, a T1 value of a heart muscle after contrast agent injection, and calculating an optimal inversion time for MRI using the T1 value of a heart muscle after contrast agent injection, based on a correlation between the ECV, the hematocrit level in blood, the T1 value of a heart muscle before contrast agent injection, the T1 value of blood before contrast agent injection, and the T1 value of blood after contrast agent injection.

In the MRI processing method according to another aspect of the present invention, in the calculating an optimal inversion time, the processor may calculate the T1 value of a heart muscle after contrast agent injection, based on Mathematical Equation 1 below:

(Mathematical Equation 1)

$$\frac{1}{T1\_Post\_Myo} = ECV \times \frac{\frac{1}{T1\_Post\_Blood} - \frac{1}{T1\_Pre\_Blood}}{1-HCT} - \frac{1}{T1\_Pre\_Myo}$$

(T1_Post_Myo: a T1 value of a heart muscle after contrast agent injection, HCT: a hematocrit level in blood, T1_Pre_Myo: a T1 value of a heart muscle before contrast agent injection, T1_Pre_Blood: a T1 value of blood before contrast agent injection, T1_Post_Blood: a T1 value of blood after contrast agent injection).

In the MRI processing method according to another aspect of the present invention, the ECV may have a value selected in the range of 24 to 28%.

In the MRI processing method according to another aspect of the present invention, the hematocrit level in blood may have a value selected in the range of 38 to 42%.

In the MRI processing method according to another aspect of the present invention, in the calculating an optimal inversion time, the processor may calculate the optimal inversion time by substituting the T1 value of a heart muscle after contrast agent injection into Mathematical Equation 2 below:

$$(\text{Mathematical Equation 2})$$

$$TI\_optimal = 0.69 \times T1\_Post\_Myo$$

(TI_optimal: optimal inversion time of MRI, T1_Post_Myo: a T1 value of a heart muscle after contrast agent injection).

The MRI processing method according to another aspect of the present invention may further include generating, by the processor, a normalized imaging signal by normalizing an imaging signal included in the MRI data at the optimal inversion time using Mathematical Equation 3 below:

$$(\text{Mathematical Equation 3})$$

$$[38] \quad S\_normal = A \times S\_TI\_optimal$$

(S_normal: normalized imaging signal, S_TI_optimal: imaging signal included in MRI data at optimal inversion time, A is a real number greater than or equal to 1).

The MRI processing method according to another aspect of the present invention may further include determining, by the processor, a portion where the normalized MRI signal is greater than or equal to a reference value on the MRI at the optimal inversion time as a lesion.

According to yet another aspect of the present invention, provided is a non-transitory computer-readable storage medium having stored thereon a program including at least one instruction for performing the MRI processing method.

ADVANTAGEOUS **EFFECT**

According to the above configuration, the MRI processing device and method, and the computer-readable recording medium storing a program for performing the method according to the present invention allow us to accurately calculate a time point when the difference in shadow between the normal tissue and the lesion is maximized on the late gadolinium enhancement (LGE) MRI, and to efficiently acquire the MRI at that time point.

Advantageous effects of the present invention are not limited to the above-described effects, and should be understood to include all effects that can be inferred from the configuration of the invention described in the detailed description or claims of the present invention.

DESCRIPTION OF DRAWINGS

FIG. 1 is a block diagram of an MRI processing device according to an exemplary embodiment of the present invention.

FIG. 2 is a diagram briefly showing the heart of a subject with a lesion in the heart muscle on a horizontal plane.

FIG. 3 is a graph showing the intensity of imaging signals of MRI of normal tissues and lesion tissues over time in a state in which a relatively greater amount of contrast agent remaining in the lesion tissue than in the normal tissue after the contrast agent is injected.

FIG. 4 is a flowchart of an MRI processing method according to an exemplary embodiment of the present invention.

MODES OF THE INVENTION

Hereinafter, exemplary embodiments of the present invention will be described in detail so that those of ordinary skill in the art can readily implement the present invention with reference to the accompanying drawings. The present invention may be embodied in many different forms and is not limited to the embodiments set forth herein. In the drawings, parts unrelated to the description are omitted for clarity of description of the present invention, and throughout the specification, same or similar reference numerals denote same elements.

Terms and words used in the present specification and claims should not be construed as limited to their usual or dictionary definition. They should be interpreted as meaning and concepts consistent with the technical idea of the present invention, based on the principle that inventors may appropriately define the terms and concepts to describe their own invention in the best way.

It should be understood that the terms "comprise or include" or "have" or the like when used in this specification, are intended to describe the presence of stated features, numbers, steps, operations, elements, components and/or a combination thereof but not preclude the possibility of the presence or addition of one or more other features, numbers, steps, operations, elements, components, or a combination thereof.

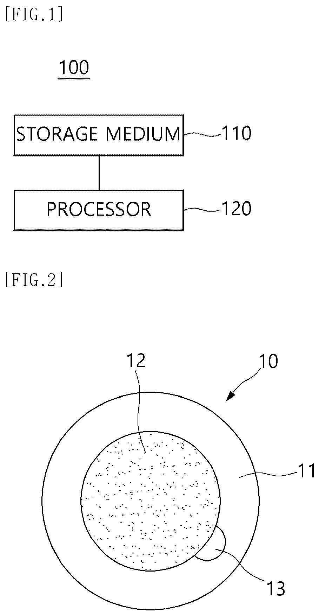

FIG. 1 is a block diagram of an MRI processing device according to an exemplary embodiment of the present invention.

The magnetic resonance image (MRI) processing device 100 according to an exemplary embodiment of the present invention is for late gadolinium enhancement magnetic resonance imaging (LGE MRI). In other words, the MRI processing device 100 according to an exemplary embodiment of the present invention is for acquiring late gadolinium enhancement (LGE) MRI using a gadolinium-based contrast agent. Hereinafter, the contrast agent may refer to a contrast agent including gadolinium.

In addition, the MRI processing device 100 according to an exemplary embodiment of the present invention may generate a T1-weighted MRI for diagnosis of an intramyocardial lesion. In more detail, the MRI processing device 100 according to an exemplary embodiment of the present invention may be for generating a synthetic late gadolinium enhancement (synLGE) image using an MRI post T1 map after contrast agent is injected.

Here, the MRI post T1 map refers to a set of MRI data that cumulatively stores a change in signal intensity for a predetermined time for each pixel of the MRI after injection of the contrast agent into the subject. More specifically, the MRI post T1 map may be defined as a set of T1 values for each pixel of the MRI.

The T1 value refers to the time it takes for a rotating proton to be inverted by an RF pulse while aligned by a magnetic field formed in an MRI device and then rearranged with respect to the magnetic field. More specifically, the T1 value may be defined as the time required for the longitudinal direction (Z-axis) component of magnetization to reach 63% of the maximum magnetization value in the initial proton alignment state after RF pulses are injected to invert the alignment of the protons in a state in which the protons aligned within the magnetic field.

In an embodiment of the present invention, the MRI post T1 map may be defined as a set of signal intensity data for each MRI pixel at an arbitrary time point in a predetermined time interval after injection of the contrast agent. In other words, the MRI post T1 map may include a set of signal intensity data for each MRI pixel at an arbitrary time point in a predetermined time interval after injection of the contrast agent.

Referring to FIG. 1, the MRI processing device 100 according to an exemplary embodiment of the present invention is an MRI processing device related to T1-weighted MRI generation and may include a storage medium 110 and a processor 120.

The storage medium 110 stores an extracellular volume (ECV), a hematocrit level in blood, a T1 value of a heart muscle before contrast agent injection, a T1 value of blood before contrast agent injection, and a T1 value of blood after contrast agent injection.

The storage medium 110 may be a magnetic medium such as a hard disk, a floppy disk and a magnetic tape; an optical medium such as a compact disk read only memory (CD-ROM) and a digital video disk (DVD); a magneto-optical medium such as a floptical disk; a read-only memory (ROM); a random access memory (RAM); a flash memory; and the like.

The ECV refers to a space or volume of tissue not occupied by cells in the human body of the subject. With respect to the myocardium of the subject, the ECV may reflect collagen content in the myocardium.

In the case of a normal person, the ECV is not affected by MRI and imaging conditions and has a constant value. Based on this fact, the ECV may be set in advance. In other words, the ECV may be determined not as a measured value, but as a preset value. For example, the ECV may have a value selected in the range of 24 to 28%. In more detail, the ECV may be determined to be 26%.

The hematocrit level in blood is expressed as a percentage of the size of the volume occupied by the red blood cells in the blood of the subject. Blood contains plasma and serum, and serum contains red blood cells, white blood cells, and platelets. The hematocrit level in blood refers to a ratio of the volume of red blood cells to the total volume of the blood.

In an embodiment of the present invention, the hematocrit level in blood may have a value measured for a subject undergoing an MRI examination.

Meanwhile, based on the fact that the measured value of the hematocrit level in blood does not differ significantly from person to person, the hematocrit level in blood may be determined as a preset value. For example, the hematocrit level in blood may have a value selected in the range of 38 to 42%. In more detail, the hematocrit level in blood may be determined to be 40%.

Regarding the T1 value of a heart muscle before contrast agent injection, the T1 value of blood before contrast agent injection, and the T1 value of blood after contrast agent injection, the T1 value refers to the time it takes for a rotating proton to be inverted by an RF pulse while aligned by a magnetic field formed in an MRI device and then rearranged with respect to the magnetic field. As described above, the T1 value may be defined as the time required for the longitudinal direction (Z-axis) component of magnetization to reach 63% of the maximum magnetization value in the initial proton alignment state after RF pulses are injected to invert the alignment of the protons in a state in which the protons aligned within the magnetic field.

Meanwhile, the T1 value of a heart muscle before contrast agent injection refers to a T1 value of a heart muscle that appears during an MRI scan in a state in which the contrast agent is not added to the subject. In addition, the T1 value of blood before contrast agent injection refers to a T1 value of blood that appears before the contrast agent is injected in the subject. In addition, the T1 value of blood after contrast agent injection refers to a T1 value of blood that appears when the contrast agent stays in the subject's blood.

In an embodiment of the present invention, measured values may be used as the T1 value of a heart muscle before contrast agent injection, the T1 value of blood before contrast agent injection, and the T1 value of blood after contrast agent injection. That is, the T1 value of a heart muscle before contrast agent injection, the T1 value of blood before contrast agent injection, and the T1 value of blood after contrast agent injection may be measured and determined for the subject.

Based on a correlation between the ECV, the hematocrit level in blood, the T1 value of a heart muscle before contrast agent injection, the T1 value of blood before contrast agent injection, and the T1 value of blood after contrast agent injection, the processor 120 calculates a T1 value of a heart muscle after contrast agent injection, and calculates an optimal inversion time for MRI using the T1 value of a heart muscle after contrast agent injection.

FIG. 2 is a diagram briefly showing the heart of a subject with a lesion in the heart muscle on a horizontal plane.

Referring to FIG. 2, the heart 10 includes a myocardium 11 and a ventricle 12 formed inside the myocardium 11. Blood may be stored in the ventricle 12. In this case, a lesion 13 may exist in the myocardium 11.

As described above, the MRI processing device 100 according to an exemplary embodiment of the present invention may be for performing late gadolinium enhancement (LGE) MRI. Late gadolinium enhancement (LGE) MRI takes advantage of the tendency of gadolinium-based contrast agents to stay longer in the intramyocardial lesion 13 than in the normal myocardium 11.

Late gadolinium enhancement MRI allows us to determine the presence or absence of a lesion based on the difference in the intensity of the imaging signal between the normal myocardium 11 and the lesion 13 shown in the MRI data obtained in the time range where most of the contrast agent in the normal myocardium 11 is discharged and a large amount of the contrast agent stays in the intramyocardial lesion 13 after the contrast agent is injected.

Synthetic late gadolinium enhancement (synLGE) MRI allows us to determine the presence or absence of a lesion based on the difference in the intensity of the imaging signal between the normal myocardium 11 and the lesion 13 shown in one or more MRI data obtained in the time range where a relatively large amount of contrast agent stays in the lesion 13 than in the normal myocardium 11 after the contrast agent is injected. In this case, the MRI data may be obtained from the above-described post T1 map. That is, the synthetic late gadolinium enhancement MRI may be derived from the post T1 map.

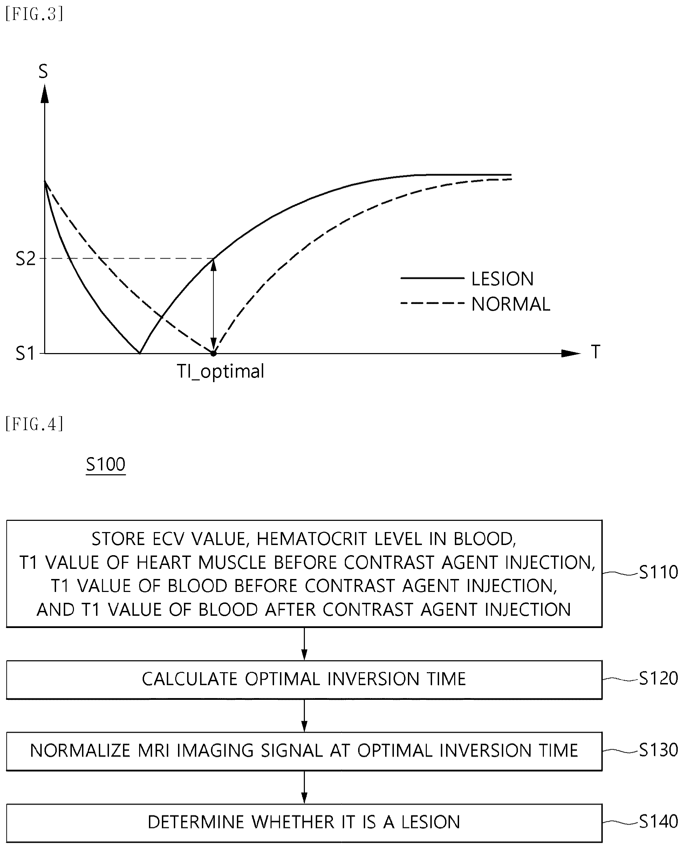

FIG. 3 is a graph showing the intensity of imaging signals of MRI of normal tissues and lesion tissues over time in a state in which a relatively greater amount of contrast agent remaining in the lesion tissue than in the normal tissue after the contrast agent is injected. More specifically, FIG. 3 is a graph showing the intensity of imaging signals of MRI of normal tissues and lesion tissues over time in a state in which most contrast agent is discharged from the normal tissue and relatively large amount of contrast agent stays in the lesion after the contrast agent is injected. In FIG. 3, the negative signal intensity is indicated by being inverted positively.

Referring to FIG. 3, the intensity of the imaging signal of the normal myocardium and the intensity of the imaging signal of the lesion included in the MRI data continuously change during the time T when, after contrast agent injection, the contrast agent in the normal myocardium 11 is discharged and the large amount of contrast agent stays in the lesion 13. When determining the presence or absence of a lesion by normalizing (or amplifying) the imaging signal of the MRI data obtained at a specific time point, it is desirable to acquire MRI data at a time point TI_optimal when the absolute value of the intensity of the imaging signal of the normal myocardium is minimal (e.g., the intensity of the imaging signal is 0) or to obtain a T1 imaging at that time point.

As shown in FIG. 3, the absolute value S2 of the intensity of the imaging signal of the lesion is greater than the intensity of the imaging signal of the normal myocardium at the time point TI_optimal when the absolute value of the intensity of the imaging signal of the normal myocardium appears as the minimum value S1 in the LGE, and when the imaging signal of the MRI data obtained at this time point (TI_optimal) is normalized (or amplified), the normal myocardium is shown dark and the lesion is shown bright on the MRI. Therefore, the presence or absence of the lesion, and furthermore, if the lesion exists, the location of the lesion can be accurately determined through MRI data at that time point.

Therefore, in an embodiment of the present invention, optimal inversion time TI_optimal may be defined as a time point when the intensity of the imaging signal of the normal myocardium included in the MRI data is minimized during the time T when a relatively large amount of contrast agent stays in the lesion 13 than in the normal myocardium 11 after the injection of the contrast agent. In other words, the optimal inversion time TI_optimal may be a time point at which the absolute value of the intensity of the normal myocardium's imaging signal included in the MRI data appears as a minimum value (e.g., 0).

In theory, the minimum value is preferably 0. However, the minimum value may be arbitrarily determined within a predetermined range in consideration of normalization (or amplification). In other words, the optimal inversion time TI_optimal may be defined as a time point arbitrarily selected within a period of time in which the intensity of the normal myocardial imaging signal appears within the predetermined range.

For example, in relation to the above minimum value, the predetermined range may be set as a range in which a value is 70 to 80 or less when the intensity of an imaging signal for a normal myocardium is normalized (or amplified). In addition, the optimal inversion time may be arbitrarily selected within a period of time in which the intensity of the imaging signal for the normal myocardium appears in the predetermined range.

With respect to the calculation of the optimal inversion time, the processor 120 may calculate the T1 value of a heart muscle after contrast agent injection based on Mathematical Equation 1 below:

(Mathematical Equation 1)

$$\frac{1}{T1\_Post\_Myo} = ECV \times \frac{\dfrac{1}{T1\_Post\_Blood} - \dfrac{1}{T1\_Pre\_Blood}}{1-HCT} - \frac{1}{T1\_Pre\_Myo}$$

(T1_Post_Myo: a T1 value of a heart muscle after contrast agent injection, HCT: a hematocrit level in blood, T1_Pre_Myo: a T1 value of a heart muscle before contrast agent injection, T1_Pre_Blood: a T1 value of blood before contrast agent injection, T1_Post_Blood: a T1 value of blood after contrast agent injection).

As will be discussed later, a T1 value of a heart muscle after contrast agent injection is required to calculate the optimal inversion time. Here, the heart muscle refers to normal tissue, and the T1 value of a heart muscle after contrast agent injection refers to a T1 value of a heart muscle that appears when the contrast agent is injected and stays in the heart muscle. As described above, the T1 value may be defined as the time until the average magnetization of 63% of the initial state is restored in the longitudinal direction after the RF pulse is injected to inverse the alignment of the protons.

The time a contrast agent stays in a heart muscle after the contrast agent is injected is very short, around a few seconds. Therefore, it is practically very difficult to measure the T1 value of a heart muscle after contrast agent injection.

In an embodiment of the present invention, the processor 120 calculates the T1 value of a heart muscle after contrast agent injection through Mathematical Equation 1 above. Accordingly, according to the present invention, it is possible to accurately calculate the T1 value of a heart muscle after contrast agent injection, which is practically very difficult to measure, and as described below, the optimal inversion time can be accurately calculated.

The processor 120 may calculate the optimal inversion time by substituting the T1 value of a heart muscle after contrast agent injection into Mathematical Equation 2 below:

(Mathematical Equation 2)

$$TI\_optimal = 0.69 \times T1\_Post\_Myo$$

(TI_optimal: optimal inversion time, T1_Post_Myo: a T1 value of a heart muscle after contrast agent injection).

In addition, the processor 120 generates a normalized imaging signal by normalizing an imaging signal included in the MRI data at the optimal inversion time TI_optimal using Mathematical Equation 3 below. More specifically, the processor 120 may select MRI data at the optimal inversion time TI_optimal among MRI data included in the post T1 map and normalize each pixel-specific imaging signal of the corresponding MRI data using Mathematical Equation 3, respectively.

(Mathematical Equation 3)

$$S\_normal = A \times S\_TI\_optimal$$

(S_normal: normalized imaging signal, S_TI_optimal: imaging signal included in MRI data at optimal inversion time, A is a real number greater than or equal to 1).

In this case, A in Mathematical Equation 3 may be determined according to the required normalization level of the signal. For example, A may be 1000. Of course, this is exemplary, and A may be increased or decrease as much as possible according to the required normalization (or amplification) level.

The absolute value of the MRI imaging signal for the normal myocardium in the process of restoring the alignment of the protons after the RF pulse is injected to inverse the alignment of the protons while the protons are aligned in the magnetic field is according to Mathematical Equation 4 below.

$$(\text{Mathematical Equation 4})$$

$$S = 1 - 2 \times e^{\frac{TI}{T1\_Post\_Myo}}$$

(TI: arbitrary time, T1_Post_Myo: a T1 value of a heart muscle after contrast agent injection)

When the optimal inversion time TI_optimal is substituted into the **TI** of Mathematical Equation 4 above, the intensity of the signal is expressed as 0. Therefore, when the processor 120 normalizes (or amplifies) imaging signal (absolute value of the intensity of the imaging signal) included in the MRI data at the optimal inversion time, the signal intensity of the normal myocardial part is not substantially amplified, while the signal intensity of the lesion part is expressed relatively high. Accordingly, on the MRI data obtained from the post T1 map, the normal myocardial part may appear dark and the lesion part may appear bright.

In addition, the processor 120 may determine the part where the normalized MRI signal appears greater or equal to a reference value on the MRI data at the optimal inversion time TI_optimal as a lesion. If there is no lesion in the heart of the subject, even if MRI data at the optimal inversion time TI_optimal is selected from among MRI data included in the post T1 map, and the imaging signal included in the corresponding MRI data is normalized, an area in which the imaging signal is greatly amplified may not appear. In consideration of this point, the processor 120 may determine a portion of the MRI data in which the normalized MRI signal is greater than or equal to a reference value as a lesion.

In this case, the reference value may be appropriately determined according to an MRI device, a photographing environment, etc. For example, the reference value may be selected from 150 to 250. In more detail, the reference value may be 200.

Meanwhile, the reference value may be stored in the storage medium 110. In other words, the reference value may be set in advance and stored in the storage medium 110.

The processor 120 may be a hardware unit that performs calculation and control in a computer. For example, the processor 120 may include at least one arithmetic logic unit (ALU) and a processing register.

Hereinafter, an MRI processing method according to an exemplary embodiment of the present invention will be described.

FIG. 4 is a flowchart of an MRI processing method according to an exemplary embodiment of the present invention.

Referring to FIG. 4, the MRI processing method S100 according to an exemplary embodiment of the present invention is an MRI processing method for generating late gadolinium enhancement (LGE) MRI (Magnetic Resonance Imaging) and may be performed as follows. The MRI processing method according to an exemplary embodiment of the present invention may be performed by the MRI processing device according to an exemplary embodiment of the present invention.

First, the storage medium 110 stores an extracellular volume (ECV), a hematocrit level in blood, a T1 value of a heart muscle before contrast agent injection, a T1 value of blood before contrast agent injection, and a T1 value of blood after contrast agent injection in step S110.

In an embodiment of the present invention, the ECV may be determined not as a measured value, but as a preset value. For example, the ECV may have a value selected in the range of 24 to 28%. In more detail, the ECV may be determined to be 26%.

In addition, the hematocrit level in blood may be determined as a preset value. For example, the hematocrit level in blood may have a value selected in the range of 38 to 42%. In more detail, the hematocrit level in blood may be determined to be 40%.

Meanwhile, measured values may be used as the T1 value of a heart muscle before contrast agent injection, the T1 value of blood before contrast agent injection, and the T1 value of blood after contrast agent injection. That is, the T1 value of a heart muscle before contrast agent injection, the T1 value of blood before contrast agent injection, and the T1 value of blood after contrast agent injection may be measured and determined for the subject.

Details on the ECV value, the hematocrit level in blood, the T1 value of a heart muscle before contrast agent injection, the T1 value of blood before contrast agent injection, and the T1 value of blood after contrast agent injection are as described above.

Next, based on a correlation between the ECV, the hematocrit level in blood, the T1 value of a heart muscle before contrast agent injection, the T1 value of blood before contrast agent injection, and the T1 value of blood after contrast agent injection, the processor 120 calculates a T1 value of a heart muscle after contrast agent injection, and calculates an optimal inversion time for MRI using the T1 value of a heart muscle after contrast agent injection in step S120.

With respect to the calculation of the optimal inversion time, the processor 120 may calculate the T1 value of a heart muscle after contrast agent injection based on Mathematical Equation 1 below:

$(Mathematical\ Equation\ 1)$

$$\frac{1}{T1\_Post\_Myo} = ECV \times \frac{\dfrac{1}{T1\_Post\_Blood} - \dfrac{1}{T1\_Pre\_Blood}}{1-HCT} - \frac{1}{T1\_Pre\_Myo}$$

(T1_Post_Myo: a T1 value of a heart muscle after contrast agent injection, HCT: a hematocrit level in blood, T1_Pre_Myo: a T1 value of a heart muscle before contrast agent injection, T1_Pre_Blood: a T1 value of blood before contrast agent injection, T1_Post_Blood: a T1 value of blood after contrast agent injection).

As previously discussed, a T1 value of a heart muscle after contrast agent injection is required to calculate the optimal inversion time. Here, the heart muscle refers to normal tissue, and the T1 value of a heart muscle after contrast agent injection refers to a T1 value of a heart muscle that appears when the contrast agent is injected and stays in the heart muscle.

The T1 value of a heart muscle after contrast agent injection is a value that is practically difficult to measure. However, according to the present invention, the processor 120 may accurately calculate the T1 value of a heart muscle after contrast agent injection through Mathematical Equation 1 above.

The optimal inversion time may be accurately calculated using the T1 value of a heart muscle after contrast agent injection. Specifically, the processor 120 may calculate the optimal inversion time by substituting the T1 value of a heart muscle after contrast agent injection into Mathematical Equation 2 below:

$(Mathematical\ Equation\ 2)$

$$TI\_optimal = 0.69 \times T1\_Post\_Myo$$

(TI_optimal: optimal inversion time of MRI, T1_Post_Myo: a T1 value of a heart muscle after contrast agent injection).

The optimal inversion time may be defined as a time point when the intensity of the imaging signal of the normal myocardium included in the MRI data is minimized during the time T when a relatively large amount of contrast agent stays in the lesion 13 than in the normal myocardium 11 after the injection of the contrast agent. In other words, the optimal inversion time may be a time point at which the absolute value of the intensity of the normal myocardium's imaging signal included in the MRI data appears as a minimum value (e.g., 0).

As described above, in theory, the minimum value is preferably 0. However, the minimum value may be arbitrarily determined within a predetermined range in consideration of normalization (or amplification). In other words, the optimal inversion time TI_optimal may be defined as a time point arbitrarily selected within a period of time in which the intensity of the normal myocardial imaging signal appears within the predetermined range.

Next, the processor 120 generates a normalized imaging signal by normalizing an imaging signal included in the MRI data at the optimal inversion time using Mathematical Equation 3 below in step S130. More specifically, the processor 120 may normalize each pixel-specific signal of MRI data at the optimal inversion time TI_optimal among MRI data included in the post T1 map using Mathematical Equation 3, respectively.

$(Mathematical\ Equation\ 3)$

$$S\_normal = A \times S\_TI\_optimal$$

(S_normal: normalized imaging signal, S_TI_optimal: imaging signal included in MRI data at optimal inversion time, A is a real number greater than or equal to 1).

In this case, A in Mathematical Equation 3 may be determined according to the required normalization level of the signal. For example, A may be 1000. Of course, this is exemplary, and A may be increased or decrease as much as possible according to the required normalization (or amplification) level.

when the processor 120 normalizes (or amplifies) imaging signal (absolute value of the intensity of the imaging signal) included in the MRI data at the optimal inversion time, the signal intensity of the normal myocardial part is not substantially amplified, while the signal intensity of the lesion part is expressed relatively high. Accordingly, on the MRI image, the normal myocardial part may appear dark and the lesion part may appear bright.

Finally, the processor 120 determines a portion where the normalized MRI signal is greater than or equal to a reference value on the MRI at the optimal inversion time as a lesion in step S140. As previously discussed, when the processor 120 normalizes (or amplifies) imaging signal (absolute value of the intensity of the imaging signal) included in the MRI data at the optimal inversion time, the signal intensity of the normal myocardial part is expressed very low, while the signal intensity of the lesion part is expressed relatively high. Based on this fact, the processor 120 may determine a portion of the MRI data in which the normalized MRI signal is greater than or equal to a reference value as a lesion.

The present invention also provides a non-transitory computer-readable storage medium having stored thereon a program including at least one instruction for performing the MRI processing method according to an exemplary embodiment described above. In this case, the instruction may include not only machine code generated by a compiler but also higher level language code executable by a computer.

The storage medium may include a hardware device configured to store and perform program instructions such as a magnetic medium such as a hard disk, a floppy disk and a magnetic tape; an optical medium such as a compact disk read only memory (CD-ROM) and a digital video disk (DVD); a magneto-optical medium such as a floptical disk; a read-only memory (ROM); a random access memory (RAM); a flash memory; and the like.

The non-transitory computer-readable storage medium according to an exemplary embodiment of the present invention may be disposed (installed) in an MRI device and used.

According to the existing synLGE method, MRI post T1 map, a set of signal intensity data for each pixel of MRI at any time point during a predetermined time after contrast agent injection, was generated, and the examiner (doctor) had to select MRI data at the appropriate time point from the MRI post T1 map to perform a diagnosis. In other words, according to the conventional synLGE method, it is passively performed to select MRI data at an appropriate time point when the shadow difference between normal tissue and lesion in the MRI post T1 map is above a certain level. Accordingly, there may be a problem that the selection of the MRI data at an inappropriate time point is made or the selection of the MRI data takes a long time.

However, according to the present invention, the optimal inversion time, which is a time point when the intensity of a imaging signal of a normal myocardium included in MRI data is minimal during the time when a relatively large amount of contrast agent stays in a lesion than in the normal myocardium after contrast agent injection, automatically calculated. In other words, a time point at which the intensity of the normal myocardial imaging signal is minimized on the post T1 map can be automatically calculated. In addition, MRI data at the optimal inversion time are selected from the post T1 map and normalized, so that the determination of the lesion can be automatically made quickly and accurately.

Although exemplary embodiments of the present invention have been described, the idea of the present invention is not limited to the embodiments set forth herein. Those of ordinary skill in the art who understand the idea of the present invention may easily propose other embodiments through supplement, change, removal, addition, etc. of elements within the same idea, but the embodiments will be also within the idea scope of the present invention.

**Claims**

1. An MRI processing device configured to generate late gadolinium enhancement (LGE) MRI (magnetic resonance imaging), the MRI processing device comprising:

   a storage medium configured to store an extracellular volume (ECV), a hematocrit level in blood, a T1 value of a heart muscle before contrast agent injection, a T1 value of blood before contrast agent injection, and a T1 value of blood after contrast agent injection; and
   a processor configured to calculate a T1 value of a heart muscle after contrast agent injection, and calculate an optimal inversion time for MRI using the T1 value of a heart muscle after contrast agent injection, based on a correlation between the ECV, the hematocrit level in blood, the T1 value of a heart muscle before contrast agent injection, the T1 value of blood before contrast agent injection, and the T1 value of blood after contrast agent injection.

2. The MRI processing device of claim 1,

   wherein the processor is configured to calculate the T1 value of a heart muscle after contrast agent injection, based on Mathematical Equation 1 below:

(Mathematical Equation 1)

$$\frac{1}{T1\_Post\_Myo} = ECV \times \frac{\frac{1}{T1\_Post\_Blood} - \frac{1}{T1\_Pre\_Blood}}{1 - HCT} - \frac{1}{T1\_Pre\_Myo}$$

(T1_Post_Myo: a T1 value of a heart muscle after contrast agent injection, HCT: a hematocrit level in blood, T1_Pre_Myo: a T1 value of a heart muscle before contrast agent injection, T1_Pre_Blood: a T1 value of blood before contrast agent injection, T1_Post_Blood: a T1 value of blood after contrast agent injection).

3. The MRI processing device of claim 1, wherein the ECV has a value selected in the range of 24 to 28%.

4. The MRI processing device of claim 1, wherein the hematocrit level in blood has a value selected in the range of 38 to 42%.

5. The MRI processing device of claim 1,

   wherein the processor is configured to calculate the optimal inversion time by substituting the T1 value of a heart muscle after contrast agent injection into Mathematical Equation 2 below:

   (Mathematical Equation 2)

   $$TI\_optimal = 0.69 \times T1\_Post\_Myo$$

   (TI_optimal: optimal inversion time, T1_Post_Myo: a T1 value of a heart muscle after contrast agent injection).

6. The MRI processing device of claim 1,

   wherein the processor is configured to generate a normalized imaging signal by normalizing an imaging signal included in the MRI data at the optimal inversion time using Mathematical Equation 3 below:

   (Mathematical Equation 3)

   $$S\_normal = A \times S\_TI\_optimal$$

   (S_normal: normalized imaging signal, S_TI_optimal: imaging signal included in MRI data at optimal inversion time, A is a real number greater than or equal to 1).

7. The MRI processing device of claim 6, wherein the processor is configured to determine a portion where the normalized MRI signal is greater than or equal to a reference value on the MRI at the optimal inversion time as a lesion.

8. An MRI processing method for generating late gadolinium enhancement (LGE) MRI (magnetic resonance imaging), the MRI processing method comprising:

   storing, by a storage medium, an extracellular volume (ECV), a hematocrit level in blood, a T1 value of a heart muscle before contrast agent injection, a T1 value of blood before contrast agent injection, and a T1 value of blood after contrast agent injection; and
   calculating, by a processor, a T1 value of a heart muscle after contrast agent injection, and calculating an optimal inversion time for MRI using the T1 value of a heart muscle after contrast agent injection, based on a correlation between the ECV, the hematocrit level in blood, the T1 value of a heart muscle before contrast agent injection, the T1 value of blood before contrast agent injection, and the T1 value of blood after contrast agent injection.

9. The MRI processing method of claim 8,

   wherein in the calculating an optimal inversion time, the processor calculates the T1 value of a heart muscle after contrast agent injection, based on Mathematical Equation 1 below:

(Mathematical Equation 1)

$$\frac{1}{T1\_Post\_Myo} = ECV \times \frac{\dfrac{1}{T1\_Post\_Blood} - \dfrac{1}{T1\_Pre\_Blood}}{1-HCT} - \frac{1}{T1\_Pre\_Myo}$$

(T1_Post_Myo: a T1 value of a heart muscle after contrast agent injection, HCT: a hematocrit level in blood, T1_Pre_Myo: a T1 value of a heart muscle before contrast agent injection, T1_Pre_Blood: a T1 value of blood before contrast agent injection, T1_Post_Blood: a T1 value of blood after contrast agent injection).

10. The MRI processing method of claim 8, wherein the ECV has a value selected in the range of 24 to 28%.

11. The MRI processing method of claim 8, wherein the hematocrit level in blood has a value selected in the range of 38 to 42%.

12. The MRI processing method of claim 8,

wherein in the calculating an optimal inversion time, the processor calculates the optimal inversion time by substituting the T1 value of a heart muscle after contrast agent injection into Mathematical Equation 2 below:

(Mathematical Equation 2)

$$TI\_optimal = 0.69 \times T1\_Post\_Myo$$

(TI_optimal: optimal inversion time of MRI, T1_Post_Myo: a T1 value of a heart muscle after contrast agent injection).

13. The MRI processing method of claim 8,

further comprising generating, by the processor, a normalized imaging signal by normalizing an imaging signal included in the MRI data at the optimal inversion time using Mathematical Equation 3 below:

(Mathematical Equation 3)

$$S\_normal = A \times S\_TI\_optimal$$

(S_normal: normalized imaging signal, S_TI_optimal: imaging signal included in MRI data at optimal inversion time, A is a real number greater than or equal to 1).

14. The MRI processing method of claim 13, further comprising determining, by the processor, a portion where the normalized MRI signal is greater than or equal to a reference value on the MRI at the optimal inversion time as a lesion.

15. A non-transitory computer-readable storage medium having stored thereon a program including at least one instruction for performing the MRI processing method of claim 8.

[FIG.1]

100

```
┌─────────────────────┐
│  STORAGE MEDIUM     │────110
└─────────────────────┘
           │
┌─────────────────────┐
│     PROCESSOR       │────120
└─────────────────────┘
```

[FIG.2]

[FIG.3]

[FIG.4]

S100

| STORE ECV VALUE, HEMATOCRIT LEVEL IN BLOOD,<br>T1 VALUE OF HEART MUSCLE BEFORE CONTRAST AGENT INJECTION,<br>T1 VALUE OF BLOOD BEFORE CONTRAST AGENT INJECTION,<br>AND T1 VALUE OF BLOOD AFTER CONTRAST AGENT INJECTION | S110 |
|---|---|
| CALCULATE OPTIMAL INVERSION TIME | S120 |
| NORMALIZE MRI IMAGING SIGNAL AT OPTIMAL INVERSION TIME | S130 |
| DETERMINE WHETHER IT IS A LESION | S140 |

## INTERNATIONAL SEARCH REPORT

| International application No. |
| --- |
| **PCT/KR2023/017006** |

### A.    CLASSIFICATION OF SUBJECT MATTER

**A61B 5/055**(2006.01)i; **A61B 5/00**(2006.01)i; **G16H 30/20**(2018.01)i; **G16H 30/40**(2018.01)i; **G16H 50/20**(2018.01)i

According to International Patent Classification (IPC) or to both national classification and IPC

### B.    FIELDS SEARCHED

Minimum documentation searched (classification system followed by classification symbols)

A61B 5/055(2006.01); A61B 5/00(2006.01); G06N 3/04(2006.01); G06N 3/08(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 자기 공명 영상(Magnetic Resonance Imaging), 심장(heart), 조영제(contrast media), T1 값(T1 value)

### C.    DOCUMENTS CONSIDERED TO BE RELEVANT

| Category* | Citation of document, with indication, where appropriate, of the relevant passages | Relevant to claim No. |
| --- | --- | --- |
| A | KR 10-1117409 B1 (SIEMENS MEDICAL SOLUTIONS USA, INC. et al.) 13 March 2012 (2012-03-13)<br>        See paragraphs [0015]-[0016]. | 1-15 |
| A | KR 10-2382199 B1 (KOREA UNIVERSITY RESEARCH AND BUSINESS FOUNDATION) 05 April 2022 (2022-04-05)<br>        See claims 1-3. | 1-15 |
| A | KR 10-1151131 B1 (UNIVERSITY-INDUSTRY COOPERATION GROUP OF KYUNG HEE UNIVERSITY) 01 June 2012 (2012-06-01)<br>        See claim 1 and figures 2-3. | 1-15 |
| A | KR 10-2022-0030896 A (PHANTOMICS INC.) 11 March 2022 (2022-03-11)<br>        See claim 1. | 1-15 |
| A | KR 10-2022-0092685 A (PHANTOMICS INC.) 04 July 2022 (2022-07-04)<br>        See entire document. | 1-15 |

☐ Further documents are listed in the continuation of Box C.          ☑ See patent family annex.

| | | | |
| --- | --- | --- | --- |
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
| --- | --- |
| **29 January 2024** | **29 January 2024** |

| Name and mailing address of the ISA/KR | Authorized officer |
| --- | --- |
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**
Information on patent family members

International application No.

**PCT/KR2023/017006**

| Patent document cited in search report | | | Publication date (day/month/year) | Patent family member(s) | | | Publication date (day/month/year) |
|---|---|---|---|---|---|---|---|
| KR | 10-1117409 | B1 | 13 March 2012 | CN | 1692880 | A | 09 November 2005 |
| | | | | CN | 1692886 | B | 08 December 2010 |
| | | | | JP | 2005-312977 | A | 10 November 2005 |
| | | | | KR | 10-2006-0043503 | A | 15 May 2006 |
| | | | | US | 2005-0245812 | A1 | 03 November 2005 |
| | | | | US | 7809426 | B2 | 05 October 2010 |
| KR | 10-2382199 | B1 | 05 April 2022 | KR | 10-2021-0002030 | A | 07 January 2021 |
| KR | 10-1151131 | B1 | 01 June 2012 | KR | 10-2012-0035022 | A | 13 April 2012 |
| KR | 10-2022-0030896 | A | 11 March 2022 | US | 11656312 | B2 | 23 May 2023 |
| | | | | US | 2022-0065966 | A1 | 03 March 2022 |
| KR | 10-2022-0092685 | A | 04 July 2022 | KR | 10-2023-0070433 | A | 23 May 2023 |
| | | | | KR | 10-2542008 | B1 | 13 June 2023 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020220030896